# EUROPEAN PATENT SPECIFICATION

(11) **EP 3 533 017 B1**
(45) Date of publication and mention of the grant of the patent: **25.11.2026**
(21) Application number: 17864845.7
(22) Date of filing: 26.10.2017
(51) Int. Cl.: G16H 50/20, G16H 70/20, G06N 5/01

(54) **STATELESS DECISION SUPPORT ENGINE**
ZUSTANDSLOSE ENGINE ZUR ENTSCHEIDUNGSUNTERSTÜTZUNG
MOTEUR D'AIDE À LA PRISE DE DÉCISION SANS ÉTAT

(30) Priority: 31.10.2016 US 201662415199 P; 24.10.2017 US 201715792548
(43) Date of publication of application: 04.09.2019
(73) Proprietor: Varian Medical Systems, Inc., Palo Alto, California 94304 (US)
(72) Inventor: ARMSTRONG, Chad, Montreal, Québec H3H 1S9 (CA); KOHANTORABI, Farzad, Toronto, Ontario M5G 2R3 (CA); DIMITROV, Stefan, Montreal, Québec H2L 5B1 (CA); ZANDI, Mohammed Naser, Montreal, Québec H4B 1N6 (CA); ERFANI, Mostafa, Montreal, Québec H4B 2Y6 (CA); SALEH, Sadiq, Dollard-des-Ormeaux, Québec H9B 0A4 (CA); STENERSON, Travis, Montreal, Québec H3C 1V8 (CA)
(74) Representative: Foster, Mark Charles
(86) International application number: PCT/CA2017/051274
(87) International publication number: WO 2018/076112

(56) References cited:
- US-A1- 2014 297 297
- US-A1- 2015 227 701
- US-A1- 2016 203 281
- US-B1- 7 107 253
- US-B2- 8 068 993

## Description

### TECHNICAL FIELD

This application relates generally to methods and systems for providing stateless decision support.

### BACKGROUND

In clinical decision making, clinicians may integrate a huge variety of clinical data to decide what information to gather, which tests to order, how to interpret and integrate such information to draw diagnostic conclusion, and which treatments to give.

In some existing and conventional methods, clinical decision making system may use stateful evaluation. Stateful evaluation requires that the system stores previous progress through best practice clinical processes and results for every request and then reloads this data in memory when a new request arrives. However, these conventional methods and solutions have created several shortcomings and a new set of challenges. Processing patient data against best practice clinical processes (many of which are long running) to provide real-time decision support to clinicians and patients is becoming increasingly difficult to achieve. The vast amounts of patient data generated today combined with constantly evolving best practice clinical processes means traditional stateful decision support engines are no longer suitable. One problem with stateful evaluation is the complexity in maintaining the state when the underlying clinical process is modified (e.g., due to recent discoveries, insights from other treatments, etc.). The stateful evaluation system has to find a corresponding stage in the new process, which is not deterministic and may involve human intervention for every patient.

More specifically, the stateful evaluation system may have the following drawbacks. First, the stateful evaluation may have negative performance impact in the evaluation process. The evaluation for every patient may have to be restored, the state may have to be identified and, once the evaluation is complete, the state may need to be persisted again. Second, the stateful evaluation may require large amount of memory. The process state including all required objects for the given stage of evaluation need to be persisted. Third, the stateful evaluation may limit the scalability of the clinical decision support (CDS) system. In distributed environments, the requirement to load and persist a state leads to synchronization problems between engine instances. Fourth, the stateful evaluation may not be fully automatable. Depending on the changes that occurred in the underlying process model the system may not be able to resume process evaluation from its persisted state that may require a human intervention to migrate the process. Fifth, the stateful evaluation may be more demanding on existing clinical information system (CIS), which may need to be aware of current process states in order to pass appropriate identifiers.

### SUMMARY

For the aforementioned reasons, there is a desire for a more automatable and efficient system and method that would allow a server to provide stateless decision support in clinical decision making process. The use of stateless clinical decision support systems may substantially improve health care quality. Discussed herein are systems and methods for implementing a clinical process, automatically converting the clinical process into a decision tree suitable for high performance stateless evaluation, receiving patient information, and evaluating patient against the decision tree to determine the evaluation results for the patient.

In one embodiment, a method comprises receiving, by a server, a model of a clinical evaluation process from a domain expert through at least one of a first interactive user interface and an application programming interface displayed on a device associated with the domain expert, wherein the clinical evaluation process refers to one or more coded clinical data; converting, by the server, the clinical evaluation process of the model into logical expressions of a decision tree by parsing the model of the clinical evaluation process, wherein the logical expression comprises decisions rules at paths or nodes, conditional expression, outcome for positive or negative evaluation, data requirements for path traversal and auxiliary data; dynamically identifying, by the server, the coded clinical data referenced in the clinical evaluation process; receiving, by the server, the referenced coded clinical data from one or more clinical terminology databases, wherein the clinical terminology databases comprise clinical codes corresponding to various diseases and medical conditions; generating and storing, by the server, the decision tree into a decision tree database, wherein the decision tree comprises the logical expressions and the referenced coded clinical data, wherein the decision tree is corresponding to the clinical treatment identifier associated with the clinical evaluation process; wherein the decision tree supports stateless evaluation; receiving, by the server, a request from an electronic client device of a client, wherein the request comprises patient data associated with a patient; determining, by the server, a selected decision tree based on the patient data, wherein a start condition corresponding to the selected decision tree is satisfied by the patient data; evaluating, by the server, the patient against the selected decision tree to determine evaluation results by traversing the selected decision tree and applying the patient data when executing the logical expressions of the selected decision tree, wherein the evaluation results comprise recommendations, requests for additional data, reminders, alerts, and alarms; and transmitting, by the server, the evaluation results to the client through at least one of the application programming interface and a second interactive user interface to display the evaluation results on the electronic client device, wherein the evaluation results are converted to a format suitable for the electronic client device.

In another embodiment, a system comprises an electronic client device; a device associated with a domain expert; a decision tree database, one or more clinical terminology databases; and a server in communication with the databases, the electronic client device and the expert computing device, wherein the server is configured to: receive a model of a clinical evaluation process from the domain expert through at least one of a first interactive user interface and an application programming interface displayed on the device associated with the domain expert, wherein the clinical evaluation process refers to one or more coded clinical data, wherein the model comprises events, gateways, sub-processes, tasks and sequence flows, a set of nodes and conditions, and a set of outcomes when the conditions are met; convert the clinical evaluation process into logical expressions of a decision tree by parsing the model of the clinical evaluation process, wherein the logical expression comprises decisions rules at paths or nodes, conditional expression, outcome for positive or negative evaluation, data requirements for path traversal and auxiliary data; dynamically identify the coded clinical data referenced in the clinical evaluation process; receive the referenced coded clinical data from the one or more clinical terminology databases, wherein the clinical terminology databases comprise clinical codes corresponding to various diseases and medical conditions; generate and store the decision tree into the decision tree database, wherein the decision tree comprises the logical expressions and the referenced coded clinical data, wherein the decision tree is corresponding to the clinical treatment identifier associated with the clinical evaluation process; wherein the decision tree supports stateless evaluation; receive a request from a client associated with the electronic client device, wherein the request comprises patient data associated with a patient ; determine a selected decision tree based on the patient data, wherein a start condition corresponding to the selected decision tree is satisfied by the patient data; evaluate the patient against the selected decision tree to determine evaluation results by traversing the selected decision tree and applying the patient data when executing the logical expressions of the selected decision tree, wherein the evaluation results comprise recommendations, requests for additional data, reminders, alerts, and alarms; and transmit the evaluation results to the client through at least one of the application programming interface and a second interactive user interface to display the evaluation results on the electronic client device, wherein the evaluation results are converted to a format suitable for the electronic client device.

It is to be understood that both the foregoing general description and the following detailed description are exemplary and explanatory and are intended to provide further explanation of the invention as claimed.

### BRIEF DESCRIPTION OF THE DRAWINGS

The accompanying drawings constitute a part of this specification and illustrate embodiments of the subject matter disclosed herein.
**FIG. 1** illustrates a computer system for providing stateless decision support, according to an exemplary embodiment.
**FIG. 2** illustrates a flowchart depicting operational steps for proving stateless decision support, according to an exemplary embodiment.
**FIG. 3** illustrates an example of decision support system architecture diagram, according to an exemplary embodiment.
**FIG. 4** illustrates an example of clinical process service component diagram, according to an exemplary embodiment.
**FIG. 5** illustrates an example of clinical decision support (CDS) engine component diagram, according to an exemplary embodiment.
**FIG. 6** illustrates an example of a knowledge set of clinical process model, according to an exemplary embodiment.
**FIG. 7** illustrates an example of a user interface to require missing patient information, according to an exemplary embodiment.
**FIG. 8** illustrates an example of a user interface with evaluation results, according to an exemplary embodiment.

### DETAILED DESCRIPTION

Reference will now be made to the illustrative embodiments illustrated in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one ordinarily skilled in the relevant art and having possession of this disclosure, are to be considered within the scope of the subject matter disclosed herein. The present disclosure is here described in detail with reference to embodiments illustrated in the drawings, which form a part here. The illustrative embodiments described in the detailed description are not meant to be limiting of the subject matter presented here.

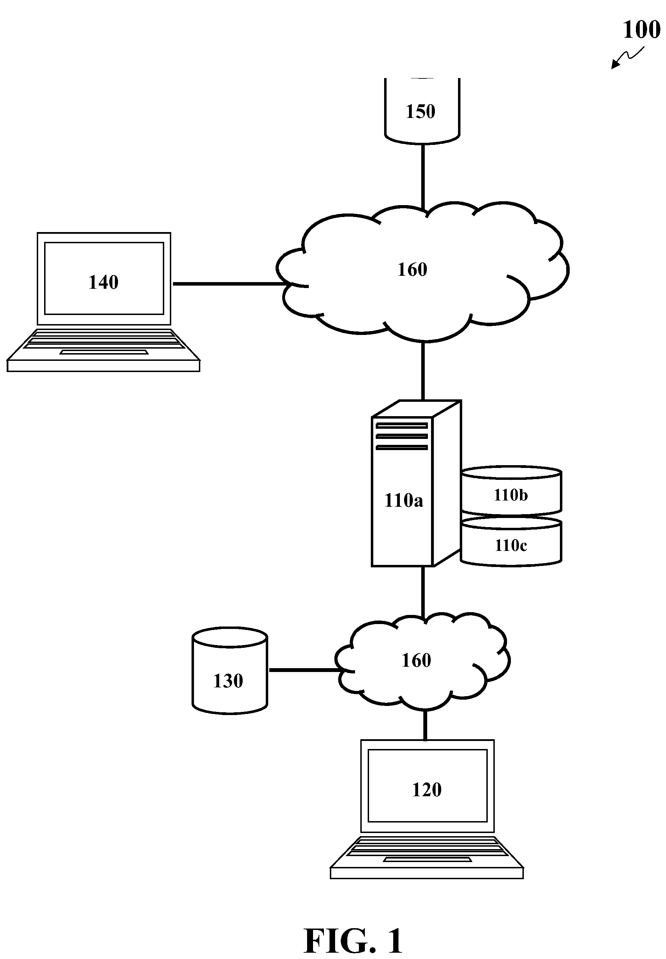

**FIG. 1** illustrates components of an exemplary system **100** for providing stateless decision support, according to an exemplary embodiment. The exemplary system **100** may comprise an analytic server **110a** with a knowledge set database **110b** and a decision tree database **110c,** an electronic client device **120,** a patient database **130,** an expert computing device **140,** a clinical terminology database **150** that are connected with each other via hardware and software components of one or more networks **160.** Examples of the network **160** include, but are not limited to, Local Area Network (LAN), Wireless Local Area Network (WLAN), Metropolitan Area Network (MAN), Wide Area Network (WAN), and the Internet. The communication over the network **160** may be performed in accordance with various communication protocols, such as Transmission Control Protocol and Internet Protocol (TCP/IP), User Datagram Protocol (UDP), and IEEE communication protocols.

The electronic client device **120** may be any computing device allowing a user to interact with analytic server **110a.** One having ordinary skill in the art would appreciate that the electronic client device **120** may be computing device comprising a processor and non-transitory machine-readable storage medium allowing the electronic client device **120** to perform the various tasks and processes when interacting with the analytic server **110a.** Examples of the computing device may include, but are not limited to, a desktop computer, a laptop, a personal digital assistant (PDA), a smartphone, a tablet computer, and the like.

The electronic client device **120** may execute an Internet browser or local application that accesses the analytic server **110a** in order to issue requests or instructions. The electronic client device **120** may transmit credentials from client inputs to the analytic server **110a,** from which the analytic server **110a** may authenticate the client and/or determine a client role. One having ordinary skill in the art would appreciate that the electronic client device **120** may comprise any number of input devices configured to receive any number of data inputs, including various types of data inputs allowing for authentication (e.g., username, passwords, certificates, and biometrics).

The electronic client device **120** may be configured to communicate with the analytic server **110a** through one or more networks **160,** using wired and/or wireless communication capabilities. In operation, the electronic client device **120** may execute a stateless decision support program, which may include a graphical user interface (GUI) that renders an interactive layout, schematic, or other elements for the client to input a request. For example, the user interface may include a text-based interface allowing the client to enter manual commands. The client may be a clinician, or other requesting entities, such as stakeholders. The request inputted by the client (e.g., clinician) may request the analytic server **110a** to make a stateless clinical decision for a patient. The electronic client device **120** may include a clinical information system (CIS) that collects patients' data and issues request to the analytic server **110a.** The patient data collected may be stored in the patient database **130.** In one or more embodiments, the clinician (operating the electronic client device **120**) may access the patient data from the patient database **130** and issue a request through the clinical information system by providing the patient's clinical data and requesting the analytic server **110a** to do a clinical evaluation based on the provided patient data.

The patient database **130** may be any non-transitory machine-readable media configured to store data, including records of relevant information for different patients. For example, the patient database **130** may include a large number of clinical statements, such as clinical test results, previous diagnosis, DNA sequence, family history and any other relevant information about each patient. One having ordinary skill in the art would appreciate that the patient database **130** may include other related data about the patient that may be used to understand patient's health situation. Different clinical organizations may have different patient data for the same patient. In operation, the patient database **130** may be logically and physically distributed across any number of physical structures and locations. For example, the patient database **130** may comprise multiple databases associated with multiple clinical organizations. In some embodiments, the clinician of a clinical organization may transmit the patient data to the analytic server **110a** when issuing a clinical evaluation request. In other embodiments, the analytic server **110a** may receive the authorization from the clinical organizations to access the patient databases. In one or more embodiments, the analytic server **110a** may need to access patient data from multiple databases associated with different clinical organizations to perform the clinical evaluation. The analytic server **110a** may convert patient record into an appropriate format. In some embodiments, the analytic server **110a** may create its own database such as a patient record database (not shown) to save the patient data received from the electronic client device **120** and/or retrieved from the patient database **130,** the evaluation results, and other related information for clinical evaluation.

The analytic server **110a** may be any computing device comprising a processor and other computing hardware and software components, configured to process the requests received from the electronic client device **120.** The analytic server **110a** may be logically and physically organized within the same or different devices or structures and may be distributed across any number of physical structures and locations (e.g., cabinets, rooms, buildings, cities). The analytic server **110a** comprises a clinical decision support (CDS) engine. Upon the analytic server **110a** receiving a request from the electronic client device **120,** the CDS engine of the analytic server **110a** may execute one or more component software models to make a clinical decision. Specifically, the analytic server **110a** may query the patient data, determine a decision tree for the clinical process, evaluate the patient against the decision tree, and determine the evaluation results. The analytic server **110a** may receive patient data provided by the clinician (operating the electronic client device **120**) when the electronic client device **120** issues a request to evaluate a specific patient. The analytic server **110a** may need to generate decision trees suitable for stateless evaluation. To generate a decision tree, the analytic server **110a** may receive clinical evaluation process using a modeling system. The analytic server **110a** may provide tools to a domain expert (operating the expert computing device **140**) to model the clinical evaluation process.

The expert computing device **140** may be any computing device comprising a processor and non-transitory machine-readable storage medium allowing the expert computing device **140** to interact with the analytic server **110a.** The examples of the expert computing device **140** may include, but are not limited to, a desktop computer, a laptop, a personal digital assistant (PDA), a smartphone, a tablet computer, and the like. One having ordinary skill in the art would appreciate that the expert computing device **140** may comprise any number of input and output devices supporting various types of data, such as text, image, audio, video, and the like. In operation, the domain expert (operating the expert computing device **140**) may have rich knowledge in a specific field and may create a knowledge set by authoring the clinical evaluation process for the specific field. For example, the domain expert may specialize in breast cancer care, and create the guideline for breast cancer evaluation using the modeling system. In operation, the domain expert may use the tools provided by the analytic server **110a** (displayed on the expert computing device **140**) to define/model the knowledge set of the clinical evaluation process.

After the analytic server **110a** receives the knowledge set from the domain expert, the analytic server may save the knowledge set into knowledge set database **110b.** The analytic server **110a** may traverse the modeled process of the knowledge set and convert it into a decision tree suitable for stateless evaluation. The modeled process of knowledge set may refer to data from other external database such as clinical terminology database **150.** In the process of generating the decision tree, the analytic server **110a** dynamically contacts external systems and databases (e.g., clinical terminology database **150**) to retrieve data referenced in the process definition. The analytic server may save the generated decision tree into a decision tree database **110c.**

The knowledge set database **110b** may be any non-transitory machine-readable media configured to store knowledge set, including the clinical evaluation processes and diagnosing processes for various diseases, authored by the domain experts in different fields. The decision tree database **110c** may be any non-transitory machine-readable media configured to store decision tree data that is the appropriate logical expressions generated by parsing the process models of the clinical evaluation processes. The analytic server **110a** may comprise or may be in network-communication with the knowledge set database **110b** and the decision tree database **110c.** The knowledge set for a certain disease may need updates when the clinical evaluation process and/or diagnosing process for a disease changes. In one or more embodiments, an update of the knowledge set database may automatically trigger update of the corresponding decision tree and the evaluation process for patients in order to provide more accurate evaluation results. In other words, when the clinical/diagnosing process for a certain disease changes, the system may update the decision tree by traversing the new clinical/diagnosing process, and evaluate patients against the new decision tree to provide real-time update of evaluation results. The knowledge set of clinical evaluation process may refer data from other database such as the clinical terminology database **150.**

The clinical terminology database **150** may be any non-transitory machine-readable media configured to store the clinical data for diagnosing various diseases. The clinical terminology database **150** may be multiple databases associated with different organizations. The analytic server **110a** may have the authorization to access the clinical terminology database **150.** For example, the analytic server **110a** may request that different organizations (database owners) provide login password or authorization tokens associated with the clinical terminology database **150.** One having ordinary skill in the art would appreciate that the analytic server **110a** may obtain authorization using any other authentication methods. In one or more embodiments, the clinical terminology database **150** may have different identifiers or codes for diseases, treatment objects, medical terminologies, and the like. The analytic server **110a** calls clinical terminology service to resolve the list of identifiers/codes that make up the various coded clinical data referenced in the clinical evaluation process.

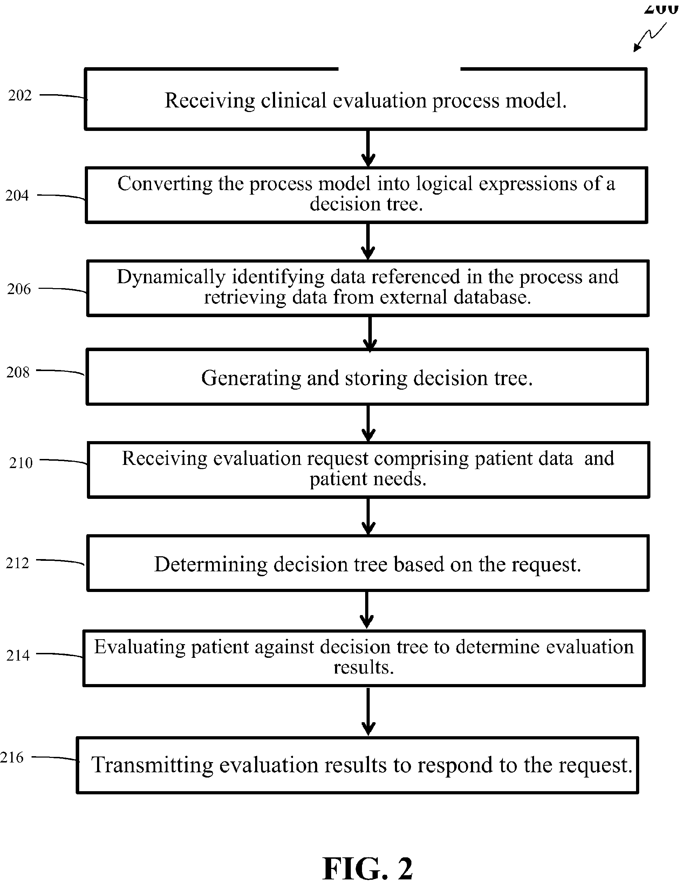

**FIG. 2** illustrates execution of an exemplary method **200** for flowchart depicting operational steps for proving stateless decision support, according to an exemplary embodiment. One having ordinary skill in the art would appreciate that other embodiments may comprise additional or alternative steps, or may omit some steps altogether.

At step **202,** the analytic server may receive clinical evaluation process model. The analytic server may generate a graphical user interface displayed on the expert computing device for the domain expert to input the clinical evaluation process model. The clinical evaluation process may be associated with a clinical treatment identifier, such as a treatment for breast cancer or diabetes. As discussed above, the domain expert may create a knowledge set by authoring the clinical evaluation process for a certain disease (e.g., breast cancer, diabetes). The process model includes but is not limited to events, gateways, sub-processes, tasks, and sequence flows. The modeling system may include elements such as sequence flows, various types of nodes and conditions, a Domain Specific Language, or another process notation. The description of a clinical evaluation process may have arbitrary number of conditions for each element and outcomes recommended if all conditions are met. The analytic server may represent the conditions and outcomes using expression language. Process components, such as gateways, may determine the order/parallelism of evaluation. The modeling system may support processes that impose requirements on the completion of certain paths in the process before further action is taken. The analytic server may store knowledge set of the modeled clinical process to the knowledge set database.

At step **204,** the analytic server may convert the process model into logical expressions of a decision tree. After receiving the clinical evaluation process authored by the domain expert, the analytic server may parse the process model and generate appropriate logical expressions of a decision tree suitable for stateless evaluation, which may include one or more decisions rules at paths or nodes, conditional expression, outcome for positive/negative evaluation, data requirements for path traversal and auxiliary data used at the evaluation time.

At step **206,** the analytic server may dynamically identify data referenced in the process and retrieve data from external database. Because the modeled clinical evaluation process may refer to data from external database such as clinical terminology database, the analytic server may dynamically identify such data and contact external database (e.g., clinical terminology database) to retrieve data referenced in the process definition. The analytic server may dynamically format and optimize the data for evaluation, so that the data is readily available at evaluation time. For example, the analytic server may contact a clinical terminology service that resolves domain-specific data elements. Dynamically identifying the data referenced in the clinical evaluation process at conversion time may allow the analytic server to complete the evaluation process in one traversal step. The CDS engine included in the analytic server may also include one or more external logic evaluation components to extend conditional expression evaluation (which may be performed by the engine kernel), thus allowing decision of arbitrary complexity to be evaluated without impacting the performance when evaluating self-contained expressions. External components may process cases such as conversion between units or references to additional data that is available only at evaluation time either within the system or queried from external systems and databases.

At step **208,** the analytic server may generate and store the decision tree. After the analytic server converts the clinical evaluation process to decision tree logic expressions and receives referenced data from external databases, the final result may be a decision tree suitable for stateless evaluation. The decision tree is corresponding to the clinical treatment identifier associated with the clinical evaluation process. The analytic server may store the decision tree into the decision tree database. The decision tree database may include a number of decision trees with each corresponding to clinical treatment identifier and/or a clinical evaluation/diagnosing process for a disease or other medical conditions.

At step **210,** the analytic server may receive an evaluation request comprising a requested treatment, patient data and patient needs. In one or more embodiments, the analytic server may provide a user interface or application programming interface (API) in the electronic client device for the client/user to issue an evaluation request. For example, the analytic server may provide an interface for internal clinical information system (CIS) or external CIS running on the electronic client device to issue requests to perform real-time or near real-time and scheduled decision support evaluations. The analytic server may receive the evaluation requests in different circumstances. For example, when the CIS needs to know the result of evaluating one or more patient records against clinical processes, the CIS may make a request to the clinical decision support (CDS) engine/service of the analytic server. As another example, the analytic server may automatically trigger the evaluation request when the patient record changes, when the clinical process changes, or when a certain amount of time has elapsed that would affect the evaluation, in order to provide real-time updates.

The received request may include treatment or clinical process requested. For example, the request may request that the analytic server evaluate one or more medical conditions for a patient, such as evaluating breast cancer or diabetes for a patient based on the patient data. In addition, the request may comprise patient data including clinical test results, previous diagnosis, DNA sequence, family history and any other relevant information about the patient that may be useful for the clinical evaluation. Furthermore, the request may comprise patient needs that may include personal preferences and other information on what is important for the patient. For example, some patients may need to be able to walk during a treatment; some other patients may need to avoid any radiation. The analytic server may consider such patient needs in the evaluation process.

At step **212,** the analytic server may determine a selected decision tree for the request. Based on the requested clinical treatment, the analytic server may determine which treatment or clinical process is requested (e.g., breast cancer or diabetes) and further determine the scope of the decisions trees where the clinical treatment identifier of the decision tree matches the request treatment. For example, the request may specify it wants to check the patient against the breast cancer process to determine appropriate treatment options. The CDS engine may retrieve the breast cancer decision trees from the decision tree database. In some embodiments, there may be more than one decision tree for a clinical process (e.g., breast cancer). In some embodiments, the analytic server may determine the selected decision tree based on the patient data, such as age, clinical history, and the like. For example, the selected decision tree may have a start condition satisfied by the patient data. In addition, the analytic server may consider the patient needs when determining the decision tree or treatment.

At step **214,** the analytic server may evaluate the patient against the decision tree to determine the evaluation results. The decision support engine of the analytic server may evaluate the patient based on the information provided within the request and additional resources. As part of the evaluation process, the analytic server may determine if additional information can impact the outcome of the evaluation and provide hints to the requesting entity (e.g., client) for such information. The hints, as well as previous decisions suggested by the CDS engine, and actions taken by the requesting system in response to decisions or hints, may be available as part of every subsequent request, and may be stored as part of the patient record. For example, the analytic server may create a separate database and store such patient record information in the separate database. One having ordinary skill in the art would appreciate that the analytic server may store the patient record data in any other systems/databases. Saving all the patient record data into a separate database may allow any instance of the CDS engine to evaluate a given request, irrespective of whether previous requests were evaluated by other instances. Additional data resources can also be replicated and geographically distributed as needed to enable the scalability and availability of the CDS engine, because synchronization between engines is not necessary.

The analytic server may convert the request and associated data records to a proper data format specific to the CDS and optimized for stateless expression evaluation. Similarly, the analytic server may convert responses from the CDS to other formats as required by the application/system (e.g., CIS) on the electronic client device. The analytic server may perform all conversions automatically without human intervention.

If a domain expert changes the underlying clinical evaluation process, the stateless CDS engine of analytic server may evaluate the patient against the newest version of the process. This is possible because all required information may be provided as part of the request, thus, the evaluation is independent of previous evaluation instances. External systems integrating with the CDS service do not need to keep track of the process states, as long as they send one or more patient records, or references to patient records, and optionally a reference to the required process version (if no version is specified, the system may verify the patient against all eligible process versions). The evaluation method may also enable hypothetical decision making by altering an instance of the patient record for one evaluation request without impacting other requests for the same patient. The system may automatically transit to an updated version of the clinical evaluation.

To further optimize the performance of CDS evaluation engine of analytic server, a response with evaluation results may include validity time. The CDS engine may automatically determine the validity time information based on the use of time-sensitive conditions in the process definitions. The CDS engine may provide the time an outcome result expires. The CDS service or a caching layer provided by the analytic server may use such expiry time upon receiving a request for evaluation to decide whether the evaluation process needs to be triggered or the previous response is still valid. If the previous response is valid, the analytic server may return the response immediately. If the underlying process definition changes, the analytic server may purge cached responses from the evaluation result cache or database and evaluate the subsequent requests based on the updated version of the process definition.

By evaluating the patient against the decision tree, the analytic server may traverse the selected decision tree and apply the patient data when executing the logical expressions of the decision tree to determine the evaluation results. The evaluation results may indicate whether the patient's treatment deviates from the clinical process, or the evaluation results may offer recommendations, requests for additional data, reminders, alerts, alarms, or other useful information to stakeholders or other CIS. The evaluation results may also be a request for more information to be provided by a person or other CIS. The stateless nature of the CDS enables real-time evaluation on patient, process or time change, in contrast to traditional stateful decision engines which may require migrating state between process versions.

At step **216,** the analytic server may transmit the evaluation results as response to the request. After the analytic server determines the evaluation results based on the patient data and the decision tree, the analytic server may store the evaluation results into a separate patient record database. Furthermore, the analytic server may transmit the evaluation results as a response to the electronic client device of the requesting entity. The analytic server may convert the evaluation results into a format suitable for the electronic client device and generate an interactive user interface to display the evaluation results.

In one or more embodiments, the analytic server may track the feedback from the patients regarding the evaluation results. For example, the analytic server may receive statistical data of feedback from a group of patients. The analytic server may identify the outcomes of clinical processes that have the most positive feedback, and update the clinical evaluation process and the corresponding decision tree to reflect the feedback associated with the evaluation results. As a result, the analytic server may be able to improve the evaluation performance.

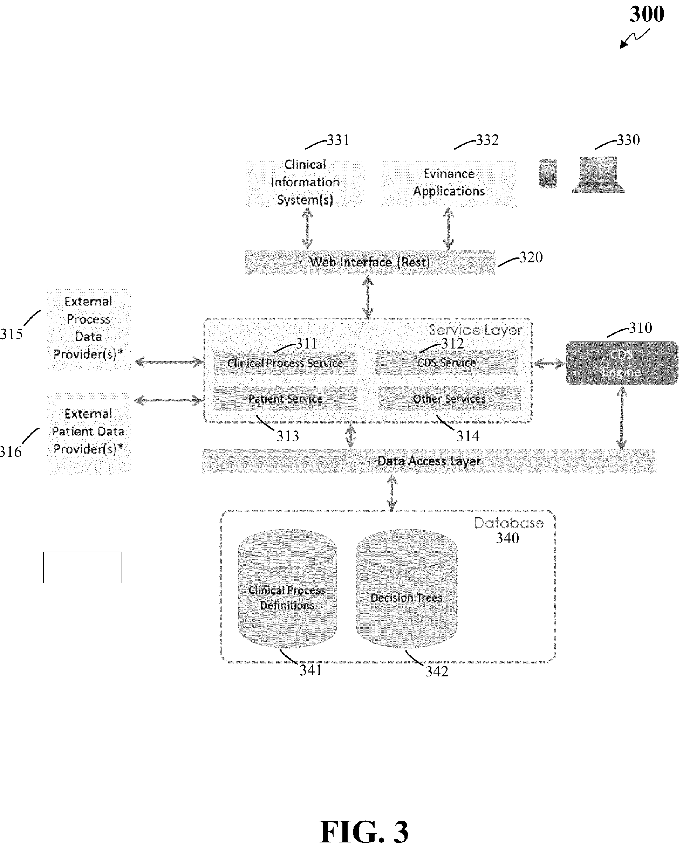

**FIG. 3** illustrates an example of decision support system architecture diagram, according to an exemplary embodiment. The system may include an analytic server with the CDS engine **310** that may provide clinical process service **311,** CDS service **312,** patient service **313,** and other services **314.** The analytic server may provide user interface **320** for the client to issue requests on the electronic client device **330** through the programs and applications (e.g., clinical information systems **331,** and internal application **332**) running on the electronic client device **330.** The CDS engine **310** may process the received request by evaluating the patient against the decision tree. To perform the evaluation, the CDS engine **310** may use clinical process service **311** to model the clinical evaluation process based on the domain expert's input and convert the process model into decision tree; use the patient service **313** to receive the patient data or related information useful for the evaluation; use the CDS service **312** to evaluate the patient against the decision tree to determine the evaluation results. In the meantime, the CDS engine **310** may access the clinical process definition database **341** to store, update, retrieve the clinical process; and access the decision tree database **342** to store, update, and retrieve the decision tree in the evaluation process. In some embodiments, the CDS engine **310** may also contact external process data providers **315** to receive the data referenced in the clinical process. In addition, the CDS engine **310** may automatically identify the missing data of patient and contact external patient data providers **316** to receive the missing patient data.

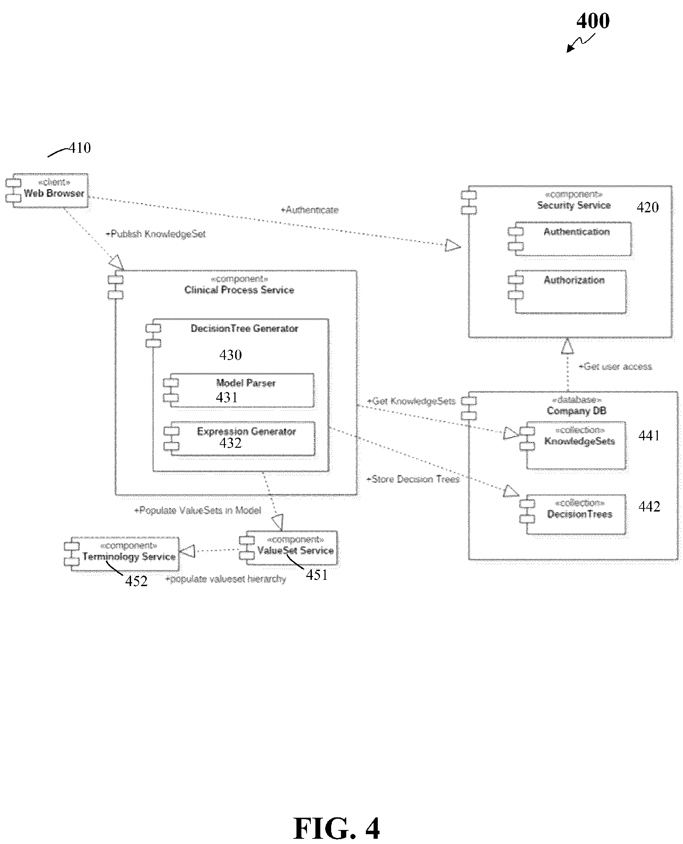

**FIG. 4** illustrates an example of clinical process service component diagram, according to an exemplary embodiment. The clinical process service provided by the analytic server may display a user interface (e.g., in web browser) **410** for the domain expert to input the clinical evaluation process. The domain expert may need to go through the authentication and authorization process in security service **420** to be able to access the tools and resources for modeling the clinical evaluation process. The inputted knowledge set of the clinical evaluation process may be stored in a knowledge set database. After the domain expert publishes the knowledge set of the clinical evaluation process, the clinical process service may trigger the decision tree generator **430.** The decision tree generator **430** includes a model parser **431** that may traverse the process model and parse the process model. An expression generator **432** that may convert the process model into logical expressions of a decision tree. Furthermore, the CDS engine may access the knowledge database **441** to get the knowledge set. In the process of converting the process model to a decision tree, the CDS engine uses value set service **451** to get value sets from the clinical terminology service **452.** For example, the clinical evaluation process may reference data from different databases; the clinical terminology service gets coded clinical data from a clinical terminology database to populate the value sets referenced in the process model. In addition, different systems/databases may use different terminologies. The terminology service **452** allows the CDS engine to resolve the various coded clinical data referenced. The final converted decision tree may be stored into a decision tree database **442.**

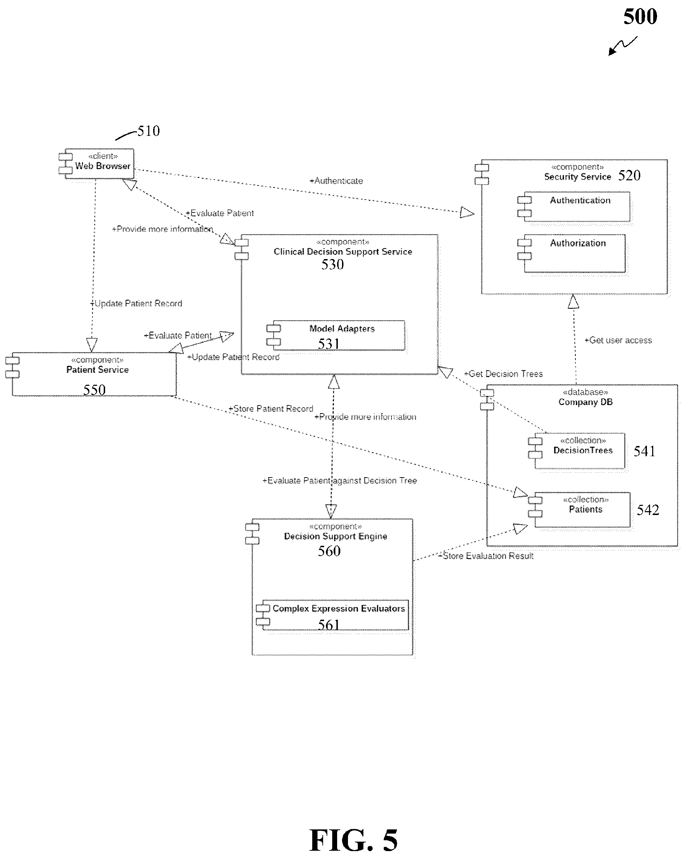

**FIG. 5** illustrates an example of CDS engine component diagram, according to an exemplary embodiment. The client may issue an evaluation request though a user interface (e.g., in web browser) **510.** The client may need to go through the authentication and authorization process in security service **520.** The CDS service **530** provided by the CDS engine may identify the decision tree from the decision tree database **541** based on the request. The CDS service of CDS engine may also receive the patient data from the patient service **550,** and store the patient data in a separate patient record database **542.** The client (e.g., a clinician) may update the patient data record. The patient service **550** may provide the updated patient record to the CDS service. The model adapters **531** may convert the patient record to appropriate format. In the evaluation process, the CDS engine may determine the missing patient data and require the client to provide more information. Based on the decision tree and the patient data from the CDS service **530,** the decision support engine **560,** which may include complex expression evaluators **561,** may evaluate the patient against the decision tree to determine the evaluation results. The evaluation results may be stored into the patient record database **542.**

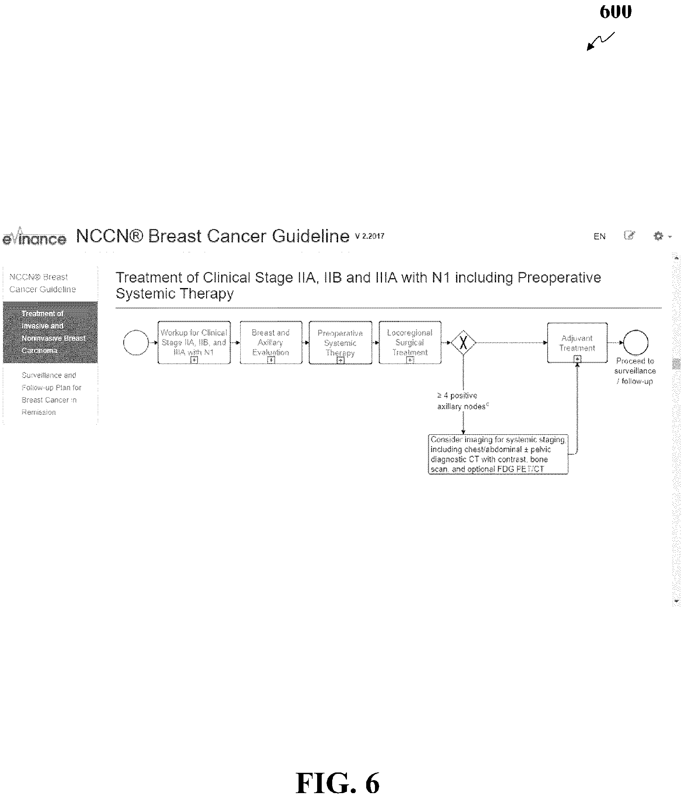

**FIG. 6** illustrates an example of a knowledge set of clinical process model, according to an exemplary embodiment. The knowledge set of the guideline may include events, gateways, sub-processes, tasks and sequence flows. For example, the clinical process model may be a breast cancer guideline authored by a domain expert. The breast cancer guideline may include different sequence flows with each one including events and gateways to evaluate the patient data and determine which flow or treatment the patient should go through.

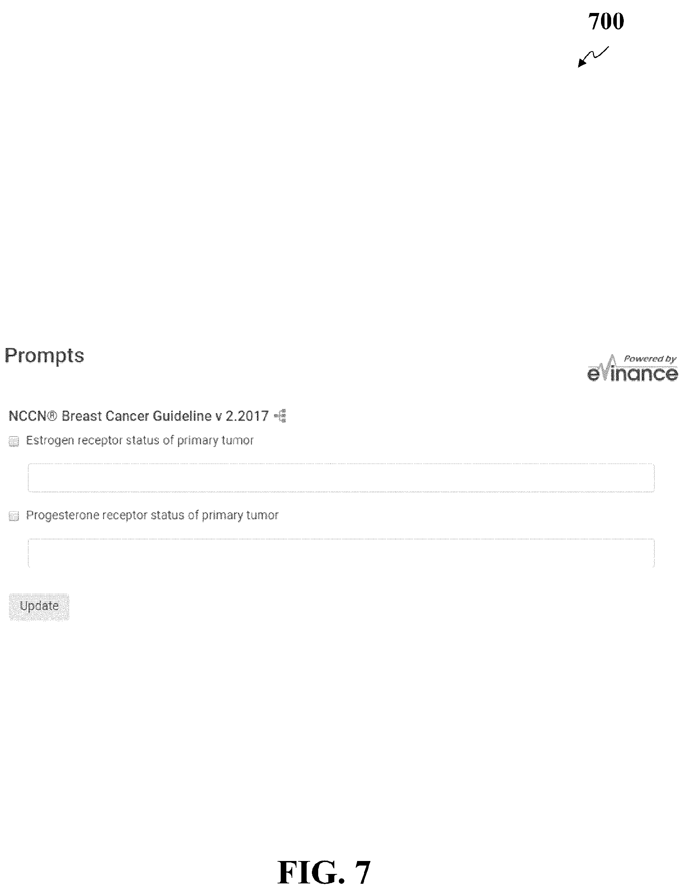

**FIG. 7** illustrates an example of a user interface to require missing patient information, according to an exemplary embodiment. In the process of evaluation, the analytic server may automatically determine the missing information of patient. For example, CDS engine may receive a request that may specify it wants to check the patient against the breast cancer process to determine appropriate treatment options. The request may comprise a patient record passed to the CDS engine through an electronic health record (EHR) system. The patient record may be in HL7 Fast Healthcare Interoperability Resources (FHIR) format that may enable exchange of healthcare-related information. Based on the request, the CDS engine may retrieve the breast cancer decision tree from the decision tree database while the model adapter converts the patient record to the appropriate format for the CDS engine. The CDS engine may use the patient data as input to traverse the breast cancer decision tree by executing the appropriate logical expressions.

In the evaluation of the breast cancer, the CDS engine may identify that the estrogen receptor (ER) status and the progesterone receptor (PR) status of the primary tumor is needed but not available as part of the patient information. The CDS engine may return a response requiring the missing information from the EHR system. The response may include a user interface for the client to provide more information (e.g., listing the ER and PR statuses specifically). The model adapter may convert the ER status and PR status observations to FHIR format and require EHR to provide more information. After the missing information (e.g., ER and PR statuses) is available, the CDS engine may reevaluate the patient.

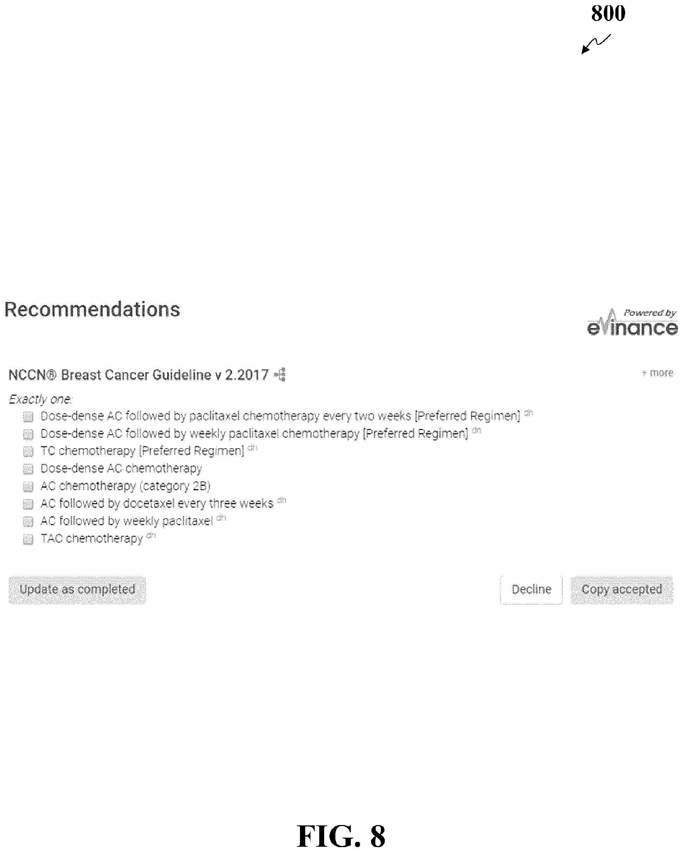

**FIG. 8** illustrates an example of a user interface with evaluation results, according to an exemplary embodiment. With the missing information available, the CDS engine may process the request in the same manner as described above and return the recommended treatment for the patient. The model adapter may convert the recommended treatment to FHIR format and display the recommendations to the client (e.g., oncologist) on the electronic client device through the EHR.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of this disclosure or the claims.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions may be stored as one or more instructions or code on a non-transitory computer-readable or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the generic principles defined herein may be applied to other embodiments Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A computer-implemented method comprising:
receiving, by a server comprising a stateless clinical decision support, CDS, engine, a model of a clinical evaluation process from a domain expert through at least one of a first interactive user interface and an application programming interface displayed on a device associated with the domain expert,
wherein the clinical evaluation process refers to coded clinical data;
wherein the model comprises events, gateways, sub-processes, tasks and sequence flows, a set of nodes and conditions, and a set of outcomes when the conditions are met;
converting, by the server, the clinical evaluation process of the model into logical expressions of a decision tree by traversing and parsing the model of the clinical evaluation process, wherein the logical expression comprises decisions rules at paths or nodes, conditional expression, outcome for positive or negative evaluation, data requirements for path traversal and auxiliary data, wherein in the process of generating the decision tree, the server dynamically contacts external systems and databases to retrieve data referenced in a process definition;
dynamically identifying, by the server, the coded clinical data referenced in the clinical evaluation process;
receiving, by the server, the referenced coded clinical data from one or more clinical terminology databases, wherein the clinical terminology databases comprise clinical codes corresponding to various diseases and medical conditions, clinical data for diagnosing various diseases, and identifiers for diseases, treatment objects and medical terminologies; wherein the server calls a clinical terminology service to resolve a list of identifiers / codes that make up the coded clinical data referenced in the clinical evaluation process;
generating and storing, by the server, the decision tree into a decision tree database,
wherein the decision tree comprises the logical expressions and the referenced coded clinical data,
wherein in the process of converting the process model to a decision tree, the CDS engine uses a value set service to get value sets from the clinical terminology service that gets coded clinical data from a clinical terminology database to populate value sets referenced in the process model, wherein the CDS engine resolves the referenced coded clinical data;
wherein the decision tree is corresponding to (i) a clinical treatment identifier associated with the clinical evaluation process and (ii) a clinical evaluation / diagnosing process for a disease or other medical conditions;
wherein the decision tree comprising logical expressions and referenced coded clinical data supports stateless evaluation, wherein evaluation is independent of previous evaluation instances;
wherein the stateless CDS engine can perform real-time evaluation without migrating state between process versions;
receiving, by the server, a request from an electronic client device of a client, wherein the request comprises patient data associated with a patient and wherein the server converts the request and associated data records to a format optimized for stateless expression evaluation;
determining, by the server, a selected decision tree based on the patient data, wherein a start condition corresponding to the selected decision tree is satisfied by the patient data;
evaluating, by the server, the patient against the selected decision tree to determine evaluation results by traversing the selected decision tree and applying the patient data when executing the logical expressions of the selected decision tree, wherein the evaluation results comprise recommendations, requests for additional data, reminders, alerts, and alarms; and
transmitting, by the server, the evaluation results to the client through at least one of the application programming interface and a second interactive user interface to display the evaluation results on the electronic client device, wherein the evaluation results are converted to a format suitable for the electronic client device.

2. The method of claim 1, further comprising:
receiving, by the server, statistic data of feedback from a group of clients associated with the evaluation results; and
updating, by the server, the decision tree to reflect the feedback associated with the evaluation results.

3. The method of claim 1, wherein the request from the client comprises patient needs,
wherein the patient needs comprise personal preferences on treatment.

4. The method of claim 3, further comprising:
determining, by the server, the selected decision tree by considering the patient needs and preferences.

5. The method of claim 1, further comprising:
automatically determining, by the server, missing information of the patient;
requesting, by the server, the missing information of the patient through at least one of the application programming interface and a user interface specifying the missing information; and
reevaluating, by the server, the patient to determine the evaluation results using the missing information when the missing information is available.

6. The method of claim 1, further comprising:
storing, by the server, the evaluation results to a patient record database.

7. The method of claim 1, further comprising:
converting, by the server, the patient data into a format desired by the server.

8. The method of claim 1, wherein the patient data comprise clinical test results, previous diagnosis, DNA sequences, and family history.

9. A system comprising:
an electronic client device;
a device associated with a domain expert;
a decision tree database, one or more clinical terminology databases; and
a server comprising a stateless clinical decision support, CDS, engine, the server in communication with the databases, the electronic client device and the expert computing device, wherein the server is configured to:
receive a model of a clinical evaluation process from the domain expert through at least one of a first interactive user interface and an application programming interface displayed on the device associated with the domain expert,
wherein the clinical evaluation process refers to coded clinical data,
wherein the model comprises events, gateways, sub-processes, tasks and sequence flows, a set of nodes and conditions, and a set of outcomes when the conditions are met;
convert the clinical evaluation process into logical expressions of a decision tree by traversing and parsing the model of the clinical evaluation process, wherein the logical expression comprises decisions rules at paths or nodes, conditional expression, outcome for positive or negative evaluation, data requirements for path traversal and auxiliary data, wherein in the process of generating the decision tree, the server dynamically contacts external systems and databases to retrieve data referenced in a process definition;
dynamically identify the coded clinical data referenced in the clinical evaluation process;
receive the referenced coded clinical data from the one or more clinical terminology databases, wherein the clinical terminology databases comprise clinical codes corresponding to various diseases and medical conditions, clinical data for diagnosing various diseases, and identifiers for diseases, treatment objects and medical terminologies;
call a clinical terminology service to resolve a list of identifiers / codes that make up the coded clinical data referenced in the clinical evaluation process;
generate and store the decision tree into the decision tree database,
wherein the decision tree comprises the logical expressions and the referenced coded clinical data,
wherein the CDS engine is configured to, in the process of converting the process model to a decision tree, use a value set service to get value sets from the clinical terminology service, which is configured to get coded clinical data from a clinical terminology database to populate value sets referenced in the process model, wherein the CDS engine is configured to resolve the referenced coded clinical data;
wherein the decision tree is corresponding to (i) a clinical treatment identifier associated with the clinical evaluation process and (ii) a clinical evaluation / diagnosing process for a disease or other medical conditions;
wherein the decision tree comprising logical expressions and referenced coded clinical data supports stateless evaluation, wherein evaluation is independent of previous evaluation instances;
wherein the stateless CDS engine is configured to perform real-time evaluation without migrating state between process versions;
receive a request from a client associated with the electronic client device, wherein the request comprises patient data associated with a patient and wherein the server converts the request and associated data records to a format optimized for stateless expression evaluation;
determine a selected decision tree based on the patient data, wherein a start condition corresponding to the selected decision tree is satisfied by the patient data;
evaluate the patient against the selected decision tree to determine evaluation results by traversing the selected decision tree and applying the patient data when executing the logical expressions of the selected decision tree, wherein the evaluation results comprise recommendations, requests for additional data, reminders, alerts, and alarms; and
transmit the evaluation results to the client through at least one of the application programming interface and a second interactive user interface to display the evaluation results on the electronic client device, wherein the evaluation results are converted to a format suitable for the electronic client device.

10. The system of claim 9, wherein the server is further configured to:
receive statistic data of feedback from a group of clients associated with the evaluation results; and
update the decision tree to reflect the feedback associated with the evaluation results.

11. The system of claim 9, wherein the request from the client comprises patient needs, wherein the patient needs comprise personal preferences on treatment.

12. The system of claim 11, wherein the server is further configured to:
determine the selected decision tree by considering the patient needs and preferences.

13. The system of claim 9, wherein the server is further configured to:
automatically determine missing information of the patient;
request the missing information of the patient through at least one of the application programming interface and a user interface specifying the missing information; and
reevaluate the patient to determine the evaluation results using the missing information when the missing information is available.

14. The system of claim 9 wherein the server is further configured to:
perform the steps of any one of claims 6 to 8.

## Patentansprüche

1. Computerimplementiertes Verfahren, umfassend:
Empfangen, durch einen Server, der eine zustandslose klinische Entscheidungsunterstützungs-, CDS-, Engine umfasst, eines Modells eines klinischen Bewertungsprozesses von einem Domänenexperten durch mindestens eine von einer ersten interaktiven Benutzerschnittstelle und einer Anwendungsprogrammierschnittstelle, die auf einer mit dem Domänenexperten assoziierten Vorrichtung angezeigt wird,
wobei der klinische Bewertungsprozess auf kodierte klinische Daten verweist;
wobei das Modell Ereignisse, Gateways, Teilprozesse, Aufgaben und Sequenzflüsse, einen Satz von Knoten und Bedingungen und einen Satz von Ergebnissen, wenn die Bedingungen erfüllt sind, umfasst;
Konvertieren, durch den Server, des klinischen Bewertungsprozesses des Modells in logische Ausdrücke eines Entscheidungsbaums durch Traversieren und Parsen des Modells des klinischen Bewertungsprozesses, wobei der logische Ausdruck Entscheidungsregeln an Pfaden oder Knoten, bedingte Ausdrücke, Ergebnisse für positive oder negative Bewertung, Datenanforderungen für Pfadtraversierung und Hilfsdaten umfasst, wobei bei dem Prozess des Erzeugens des Entscheidungsbaums der Server dynamisch externe Systeme und Datenbanken kontaktiert, um in einer Prozessdefinition referenzierte Daten abzurufen;
dynamisches Identifizieren, durch den Server, der kodierten klinischen Daten, die in dem klinischen Bewertungsprozess referenziert sind;
Empfangen, durch den Server, der referenzierten kodierten klinischen Daten von einer oder mehreren klinischen Terminologiedatenbanken, wobei die klinischen Terminologiedatenbanken klinische Codes, die verschiedenen Krankheiten und medizinischen Zuständen entsprechen, klinische Daten zum Diagnostizieren verschiedener Krankheiten und Bezeichner für Krankheiten, Behandlungsobjekte und medizinische Terminologien umfassen; wobei der Server einen klinischen Terminologiedienst aufruft, um eine Liste von Bezeichnern / Codes aufzulösen, die die kodierten klinischen Daten bilden, die in dem klinischen Bewertungsprozess referenziert sind;
Erzeugen und Speichern, durch den Server, des Entscheidungsbaums in einer Entscheidungsbaumdatenbank,
wobei der Entscheidungsbaum die logischen Ausdrücke und die referenzierten kodierten klinischen Daten umfasst,
wobei bei dem Prozess des Konvertierens des Prozessmodells in einen Entscheidungsbaum die CDS-Engine einen Wertesetdienst verwendet, um Wertesets von dem klinischen Terminologiedienst zu erhalten, der kodierte klinische Daten von einer klinischen Terminologiedatenbank erhält, um in dem Prozessmodell referenzierte Wertesets zu befüllen, wobei die CDS-Engine die referenzierten kodierten klinischen Daten auflöst;
wobei der Entscheidungsbaum (i) einem klinischen Behandlungsbezeichner, der mit dem klinischen Bewertungsprozess assoziiert ist, und (ii) einem klinischen Bewertungs-/Diagnoseprozess für eine Krankheit oder andere medizinische Zustände entspricht;
wobei der Entscheidungsbaum, umfassend logische Ausdrücke und referenzierte kodierte klinische Daten, zustandslose Bewertung unterstützt, wobei Bewertung unabhängig von vorherigen Bewertungsinstanzen ist;
wobei die zustandslose CDS-Engine Echtzeitbewertung durchführen kann, ohne Zustand zwischen Prozessversionen zu migrieren;
Empfangen, durch den Server, einer Anfrage von einer elektronischen Clientvorrichtung eines Clients, wobei die Anfrage mit einem Patienten assoziierte Patientendaten umfasst und wobei der Server die Anfrage und assoziierte Datensätze in ein für zustandslose Ausdrucksauswertung optimiertes Format konvertiert;
Bestimmen, durch den Server, eines ausgewählten Entscheidungsbaums basierend auf den Patientendaten, wobei eine Startbedingung, die dem ausgewählten Entscheidungsbaum entspricht, durch die Patientendaten erfüllt ist;
Auswerten, durch den Server, des Patienten gegen den ausgewählten Entscheidungsbaum, um Bewertungsergebnisse zu bestimmen, durch Traversieren des ausgewählten Entscheidungsbaums und Anwenden der Patientendaten beim Ausführen der logischen Ausdrücke des ausgewählten Entscheidungsbaums, wobei die Bewertungsergebnisse Empfehlungen, Anfragen nach zusätzlichen Daten, Erinnerungen, Warnhinweise und Alarme umfassen; und
Übertragen, durch den Server, der Bewertungsergebnisse an den Client durch mindestens eine von der Anwendungsprogrammierschnittstelle und einer zweiten interaktiven Benutzerschnittstelle, um die Bewertungsergebnisse auf der elektronischen Clientvorrichtung anzuzeigen, wobei die Bewertungsergebnisse in ein für die elektronische Clientvorrichtung geeignetes Format konvertiert werden.

2. Verfahren nach Anspruch 1, ferner umfassend:
Empfangen, durch den Server, statistischer Daten von Feedback von einer Gruppe von Clients, die mit den Bewertungsergebnissen assoziiert sind; und
Aktualisieren, durch den Server, des Entscheidungsbaums, um das Feedback widerzuspiegeln, das mit den Bewertungsergebnissen assoziiert ist.

3. Verfahren nach Anspruch 1, wobei die Anfrage von dem Client Patientenbedürfnisse umfasst, wobei die Patientenbedürfnisse persönliche Präferenzen bezüglich Behandlung umfassen.

4. Verfahren nach Anspruch 3, ferner umfassend:
Bestimmen, durch den Server, des ausgewählten Entscheidungsbaums durch Berücksichtigung der Patientenbedürfnisse und Präferenzen.

5. Verfahren nach Anspruch 1, ferner umfassend:
automatisches Bestimmen, durch den Server, fehlender Informationen des Patienten;
Anfordern, durch den Server, der fehlenden Informationen des Patienten durch mindestens eine von der Anwendungsprogrammierschnittstelle und einer Benutzerschnittstelle, die die fehlenden Informationen definiert; und
erneutes Auswerten, durch den Server, des Patienten, um die Bewertungsergebnisse unter Verwendung der fehlenden Informationen zu bestimmen, wenn die fehlenden Informationen verfügbar sind.

6. Verfahren nach Anspruch 1, ferner umfassend:
Speichern, durch den Server, der Bewertungsergebnisse in einer Patientenaktendatenbank.

7. Verfahren nach Anspruch 1, ferner umfassend:
Konvertieren, durch den Server, der Patientendaten in ein von dem Server gewünschtes Format.

8. Verfahren nach Anspruch 1, wobei die Patientendaten klinische Testergebnisse, vorherige Diagnosen, DNA-Sequenzen und Familienanamnese umfassen.

9. System, umfassend:
eine elektronische Clientvorrichtung;
eine mit einem Domänenexperten assoziierte Vorrichtung;
eine Entscheidungsbaumdatenbank, eine oder mehrere klinische Terminologiedatenbanken; und
einen Server, umfassend eine zustandslose klinische Entscheidungsunterstützungs-, CDS-, Engine, wobei der Server in Kommunikation mit den Datenbanken, der elektronischen Clientvorrichtung und der Expertenrechenvorrichtung steht,
wobei der Server konfiguriert ist zum:
Empfangen eines Modells eines klinischen Bewertungsprozesses von dem Domänenexperten durch mindestens eine von einer ersten interaktiven Benutzerschnittstelle und einer Anwendungsprogrammierschnittstelle, die auf der dem Domänenexperten zugeordneten Vorrichtung angezeigt wird,
wobei der klinische Bewertungsprozess auf kodierte klinische Daten verweist,
wobei das Modell Ereignisse, Gateways, Teilprozesse, Aufgaben und Sequenzflüsse, einen Satz von Knoten und Bedingungen und einen Satz von Ergebnissen, wenn die Bedingungen erfüllt sind, umfasst;
Konvertieren des klinischen Bewertungsprozesses in logische Ausdrücke eines Entscheidungsbaums durch Traversieren und Parsen des Modells des klinischen Bewertungsprozesses, wobei der logische Ausdruck Entscheidungsregeln an Pfaden oder Knoten, bedingte Ausdrücke, Ergebnisse für positive oder negative Bewertung, Datenanforderungen für Pfadtraversierung und Hilfsdaten umfasst, wobei der Server im Prozess des Erzeugens des Entscheidungsbaums dynamisch externe Systeme und Datenbanken kontaktiert, um in einer Prozessdefinition referenzierte Daten abzurufen;
dynamisches Identifizieren der kodierten klinischen Daten, die in dem klinischen Bewertungsprozess referenziert sind;
Empfangen der referenzierten kodierten klinischen Daten von den einen oder mehreren klinischen Terminologiedatenbanken, wobei die klinischen Terminologiedatenbanken klinische Codes, die verschiedenen Krankheiten und medizinischen Zuständen entsprechen, klinische Daten zum Diagnostizieren verschiedener Krankheiten, und Bezeichner für Krankheiten, Behandlungsobjekte und medizinische Terminologien umfassen;
Aufrufen eines klinischen Terminologiedienstes zum Auflösen einer Liste von Bezeichnern / Codes, die die kodierten klinischen Daten bilden, die in dem klinischen Bewertungsprozess referenziert sind;
Erzeugen und Speichern des Entscheidungsbaums in der Entscheidungsbaumdatenbank,
wobei der Entscheidungsbaum die logischen Ausdrücke und die referenzierten kodierten klinischen Daten umfasst,
wobei die CDS-Engine konfiguriert ist, im Prozess des Konvertierens des Prozessmodells in einen Entscheidungsbaum einen Wertesetdienst zu verwenden, um Wertesets von dem klinischen Terminologiedienst zu erhalten, der konfiguriert ist, kodierte klinische Daten von einer klinischen Terminologiedatenbank zu erhalten, um in dem Prozessmodell referenzierte Wertesets zu befüllen, wobei die CDS-Engine konfiguriert ist, die referenzierten kodierten klinischen Daten aufzulösen;
wobei der Entscheidungsbaum (i) einem klinischen Behandlungsbezeichner, der mit dem klinischen Bewertungsprozess assoziiert ist, und (ii) einem klinischen Bewertungs-/Diagnoseprozess für eine Krankheit oder andere medizinische Zustände entspricht;
wobei der Entscheidungsbaum, umfassend logische Ausdrücke und referenzierte kodierte klinische Daten, zustandslose Bewertung unterstützt, wobei Bewertung unabhängig von vorherigen Bewertungsinstanzen ist;
wobei die zustandslose CDS-Engine konfiguriert ist, Echtzeitbewertung durchzuführen, ohne Zustand zwischen Prozessversionen zu migrieren;
Empfangen einer Anfrage von einem Client, der der elektronischen Clientvorrichtung zugeordnet ist, wobei die Anfrage Patientendaten, die einem Patienten zugeordnet sind, umfasst, und wobei der Server die Anfrage und zugehörige Datensätze in ein für zustandslose Ausdrucksauswertung optimiertes Format konvertiert;
Bestimmen eines ausgewählten Entscheidungsbaums basierend auf den Patientendaten, wobei eine Startbedingung, die dem ausgewählten Entscheidungsbaum entspricht, durch die Patientendaten erfüllt ist;
Bewerten des Patienten anhand des ausgewählten Entscheidungsbaums zum Bestimmen von Bewertungsergebnissen durch Traversieren des ausgewählten Entscheidungsbaums und Anwenden der Patientendaten beim Ausführen der logischen Ausdrücke des ausgewählten Entscheidungsbaums, wobei die Bewertungsergebnisse Empfehlungen, Anfragen nach zusätzlichen Daten, Erinnerungen, Warnhinweise und Alarme umfassen; und
Übertragen der Bewertungsergebnisse an den Client durch mindestens eine von der Anwendungsprogrammierschnittstelle und einer zweiten interaktiven Benutzerschnittstelle zum Anzeigen der Bewertungsergebnisse auf der elektronischen Clientvorrichtung, wobei die Bewertungsergebnisse in ein für die elektronische Clientvorrichtung geeignetes Format konvertiert werden.

10. System nach Anspruch 9, wobei der Server ferner konfiguriert ist zum:
Empfangen statistischer Daten von Rückmeldungen von einer Gruppe von Clients, die den Bewertungsergebnissen zugeordnet sind; und
Aktualisieren des Entscheidungsbaums, um die den Bewertungsergebnissen zugeordnete Rückmeldung widerzuspiegeln.

11. System nach Anspruch 9, wobei die Anfrage von dem Client Patientenbedürfnisse umfasst, wobei die Patientenbedürfnisse persönliche Präferenzen bezüglich Behandlung umfassen.

12. System nach Anspruch 11, wobei der Server ferner konfiguriert ist zum:
Bestimmen des ausgewählten Entscheidungsbaums unter Berücksichtigung der Patientenbedürfnisse und Präferenzen.

13. System nach Anspruch 9, wobei der Server ferner konfiguriert ist zum:
automatischen Bestimmen fehlender Informationen des Patienten; Anfordern der fehlenden Informationen des Patienten durch mindestens eine von der Anwendungsprogrammierschnittstelle und einer Benutzerschnittstelle, die die fehlenden Informationen definiert; und
erneuten Bewerten des Patienten zum Bestimmen der Bewertungsergebnisse unter Verwendung der fehlenden Informationen, wenn die fehlenden Informationen verfügbar sind.

14. System nach Anspruch 9, wobei der Server ferner konfiguriert ist zum:
Durchführen der Schritte nach einem der Ansprüche 6 bis 8.

## Revendications

1. Procédé mis en œuvre par ordinateur comprenant :
la réception, par un serveur comprenant un moteur d'aide à la prise de décision clinique, CDS, sans état d'un modèle d'un processus d'évaluation clinique provenant d'un expert du domaine à travers au moins l'une d'une première interface utilisateur interactive et d'une interface de programmation d'application affichée sur un dispositif associé à l'expert du domaine,
dans lequel le processus d'évaluation clinique fait référence à des données cliniques codées ;
dans lequel le modèle comprend des événements, des passerelles, des sous-processus, des tâches et des flux de séquences, un ensemble de nœuds et de conditions, et un ensemble de résultats lorsque les conditions sont remplies ;
la conversion, par le serveur, du processus d'évaluation clinique du modèle en expressions logiques d'un arbre de décision en parcourant et en analysant le modèle du processus d'évaluation clinique, dans lequel l'expression logique comprend des règles de décision au niveau de chemins ou de nœuds, une expression conditionnelle, un résultat pour une évaluation positive ou négative, des exigences de données pour un parcours de chemin et des données auxiliaires, dans lequel, au cours du processus de génération de l'arbre de décision, le serveur contacte dynamiquement des systèmes et des bases de données externes pour récupérer des données référencées dans une définition de processus ;
l'identification dynamique, par le serveur, des données cliniques codées référencées dans le processus d'évaluation clinique ;
la réception, par le serveur, des données cliniques codées référencées à partir d'une ou de plusieurs bases de données de terminologie clinique, dans lequel les bases de données de terminologie clinique comprennent des codes cliniques correspondant à diverses maladies et affections médicales, des données cliniques pour le diagnostic de diverses maladies et des identifiants pour les maladies, les objets de traitement et les terminologies médicales ; dans lequel le serveur appelle un service de terminologie clinique pour résoudre une liste d'identifiants/codes qui constituent les données cliniques codées référencées dans le processus d'évaluation clinique ;
la génération et le stockage, par le serveur, de l'arbre de décision dans une base de données d'arbres de décision,
dans lequel l'arbre de décision comprend les expressions logiques et les données cliniques codées référencées,
dans lequel, au cours du processus de conversion du modèle de processus en un arbre de décision, le moteur CDS utilise un service d'ensemble de valeurs pour obtenir des ensembles de valeurs à partir du service de terminologie clinique qui obtient des données cliniques codées à partir d'une base de données de terminologie clinique pour remplir des ensembles de valeurs référencés dans le modèle de processus, dans lequel le moteur CDS résout les données cliniques codées référencées ;
dans lequel l'arbre de décision correspond (i) à un identifiant de traitement clinique associé au processus d'évaluation clinique et (ii) à un processus d'évaluation/de diagnostic clinique pour une maladie ou d'autres affections médicales ;
dans lequel l'arbre de décision comprenant des expressions logiques et des données cliniques codées référencées prend en charge une évaluation sans état, dans lequel l'évaluation est indépendante des instances d'évaluation précédentes ;
dans lequel le moteur CDS sans état peut réaliser une évaluation en temps réel sans migration d'état entre les versions de processus ;
la réception, par le serveur, d'une demande à partir d'un dispositif client électronique d'un client, dans lequel la demande comprend des données patient associées à un patient et dans lequel le serveur convertit la demande et les enregistrements de données associés en un format optimisé pour l'évaluation d'expressions sans état ;
la détermination, par le serveur, d'un arbre de décision sélectionné sur la base des données patient, dans lequel une condition de départ correspondant à l'arbre de décision sélectionné est satisfaite par les données patient ;
l'évaluation, par le serveur, du patient par rapport à l'arbre de décision sélectionné afin de déterminer les résultats de l'évaluation en parcourant l'arbre de décision sélectionné et en appliquant les données patient lors de l'exécution des expressions logiques de l'arbre de décision sélectionné, dans lequel les résultats de l'évaluation comprennent des recommandations, des demandes de données supplémentaires, des rappels, des alertes et des alarmes ; et
la transmission, par le serveur, des résultats de l'évaluation au client à travers au moins l'une de l'interface de programmation d'application et d'une seconde interface utilisateur interactive pour afficher les résultats de l'évaluation sur le dispositif client électronique, dans lequel les résultats de l'évaluation sont convertis dans un format adapté au dispositif client électronique.

2. Procédé selon la revendication 1, comprenant en outre :
la réception, par le serveur, de données statistiques sur les commentaires d'un groupe de clients associés aux résultats de l'évaluation ; et
la mise à jour, par le serveur, de l'arbre de décision pour refléter les commentaires associés aux résultats de l'évaluation.

3. Procédé selon la revendication 1, dans lequel la demande du client comprend les besoins du patient,
dans lequel les besoins du patient comprennent ses préférences personnelles en matière de traitement.

4. Procédé selon la revendication 3, comprenant en outre :
la détermination, par le serveur, de l'arbre de décision sélectionné en tenant compte des besoins et des préférences du patient.

5. Procédé selon la revendication 1 comprenant en outre :
la détermination automatique, par le serveur, d'informations manquantes du patient ;
la demande, par le serveur, des informations manquantes du patient à travers au moins l'une de l'interface de programmation d'application et d'une interface utilisateur spécifiant les informations manquantes ;
et
la réévaluation, par le serveur, du patient afin de déterminer les résultats de l'évaluation à l'aide des informations manquantes lorsque les informations manquantes sont disponibles.

6. Procédé selon la revendication 1, comprenant en outre :
le stockage, par le serveur, des résultats de l'évaluation dans une base de données de dossiers patients.

7. Procédé selon la revendication 1, comprenant en outre :
la conversion, par le serveur, des données patient dans un format souhaité par le serveur.

8. Procédé selon la revendication 1, dans lequel les données patient comprennent les résultats des tests cliniques, les diagnostics antérieurs, les séquences d'ADN et les antécédents familiaux.

9. Système comprenant :
un dispositif client électronique ;
un dispositif associé à un expert du domaine ;
une base de données d'arbres de décision, une ou plusieurs bases de données de terminologie clinique ; et
un serveur comprenant un moteur d'aide à la prise de décision clinique, CDS, sans état, le serveur étant en communication avec les bases de données, le dispositif client électronique et le dispositif informatique expert, dans lequel le serveur est configuré pour :
recevoir un modèle d'un processus d'évaluation clinique provenant de l'expert du domaine à travers au moins l'une d'une première interface utilisateur interactive et d'une interface de programmation d'application affichée sur le dispositif associé à l'expert du domaine,
dans lequel le processus d'évaluation clinique fait référence à des données cliniques codées ;
dans lequel le modèle comprend des événements, des passerelles, des sous-processus, des tâches et des flux de séquences, un ensemble de nœuds et de conditions, et un ensemble de résultats lorsque les conditions sont remplies ;
convertir le processus d'évaluation clinique en expressions logiques d'un arbre de décision en parcourant et en analysant le modèle du processus d'évaluation clinique, dans lequel l'expression logique comprend des règles de décision au niveau de chemins ou de nœuds, une expression conditionnelle, un résultat pour une évaluation positive ou négative, des exigences de données pour un parcours de chemin et des données auxiliaires, dans lequel, au cours du processus de génération de l'arbre de décision, le serveur contacte dynamiquement des systèmes et des bases de données externes pour récupérer des données référencées dans une définition de processus ;
identifier dynamiquement les données cliniques codées référencées dans le processus d'évaluation clinique ;
recevoir les données cliniques codées référencées à partir des une ou plusieurs bases de données de terminologie clinique, dans lequel les bases de données de terminologie clinique comprennent des codes cliniques correspondant à diverses maladies et affections médicales, des données cliniques pour le diagnostic de diverses maladies et des identifiants pour les maladies, les objets de traitement et les terminologies médicales ;
appeler un service de terminologie clinique pour résoudre une liste d'identifiants/codes qui constituent les données cliniques codées référencées dans le processus d'évaluation clinique ;
générer et stocker l'arbre de décision dans la base de données d'arbres de décision,
dans lequel l'arbre de décision comprend les expressions logiques et les données cliniques codées référencées,
dans lequel le moteur CDS est configuré pour, au cours du processus de conversion du modèle de processus en un arbre de décision, utiliser un service d'ensemble de valeurs pour obtenir des ensembles de valeurs à partir du service de terminologie clinique, lequel est configuré pour obtenir des données cliniques codées à partir d'une base de données de terminologie clinique pour remplir des ensembles de valeurs référencés dans le modèle de processus, dans lequel le moteur CDS est configuré pour résoudre les données cliniques codées référencées ;
dans lequel l'arbre de décision correspond (i) à un identifiant de traitement clinique associé au processus d'évaluation clinique et (ii) à un processus d'évaluation/de diagnostic clinique pour une maladie ou d'autres affections médicales ;
dans lequel l'arbre de décision comprenant des expressions logiques et des données cliniques codées référencées prend en charge une évaluation sans état, dans lequel l'évaluation est indépendante des instances d'évaluation précédentes ;
dans lequel le moteur CDS sans état est configuré pour réaliser une évaluation en temps réel sans migration d'état entre les versions de processus ;
recevoir une demande à partir d'un client associé au dispositif client électronique, dans lequel la demande comprend des données patient associées à un patient et dans lequel le serveur convertit la demande et les enregistrements de données associés en un format optimisé pour l'évaluation d'expressions sans état ;
déterminer un arbre de décision sélectionné sur la base des données patient, dans lequel une condition de départ correspondant à l'arbre de décision sélectionné est satisfaite par les données patient ;
évaluer le patient par rapport à l'arbre de décision sélectionné afin de déterminer les résultats de l'évaluation en parcourant l'arbre de décision sélectionné et en appliquant les données patient lors de l'exécution des expressions logiques de l'arbre de décision sélectionné, dans lequel les résultats de l'évaluation comprennent des recommandations, des demandes de données supplémentaires, des rappels, des alertes et des alarmes ; et
transmettre les résultats de l'évaluation au client à travers au moins l'une de l'interface de programmation d'application et d'une seconde interface utilisateur interactive pour afficher les résultats de l'évaluation sur le dispositif client électronique, dans lequel les résultats de l'évaluation sont convertis dans un format adapté au dispositif client électronique.

10. Système selon la revendication 9, dans lequel le serveur est en outre configuré pour :
recevoir des données statistiques sur les commentaires d'un groupe de clients associés aux résultats de l'évaluation ; et
mettre à jour l'arbre de décision pour refléter les commentaires associés aux résultats de l'évaluation.

11. Système selon la revendication 9, dans lequel la demande du client comprend les besoins du patient, dans lequel les besoins du patient comprennent des préférences personnelles en matière de traitement.

12. Système selon la revendication 11, dans lequel le serveur est en outre configuré pour :
déterminer l'arbre de décision sélectionné en tenant compte des besoins et des préférences du patient.

13. Système selon la revendication 9, dans lequel le serveur est en outre configuré pour :
déterminer automatiquement les informations manquantes du patient ;
demander les informations manquantes du patient à travers au moins l'une de l'interface de programmation d'application et
d'une interface utilisateur spécifiant les informations manquantes ; et
réévaluer le patient afin de déterminer les résultats de l'évaluation à l'aide des informations manquantes lorsque les informations manquantes sont disponibles.

14. Système selon la revendication 9, dans lequel le serveur est en outre configuré pour :
réaliser les étapes selon l'une quelconque des revendications 6 à 8.
